# EUROPEAN PATENT APPLICATION

(11) **EP 4 053 555 A1**
(43) Date of publication of application: **07.09.2022**
(21) Application number: 20882077.9
(22) Date of filing: 30.10.2020
(51) Int. Cl.: G01N 33/493, G01N 33/53

(54) **METHOD FOR MEASURING STEROID IN URINE SAMPLE, AND KIT FOR MEASURING STEROID IN URINE SAMPLE AND NEUTRALIZATION SOLUTION BOTH FOR USE IN SAID METHOD**

(30) Priority: 01.11.2019 JP 2019199808
(71) Applicant: Fujirebio Inc., Shinjuku-ku Tokyo 163-0410 (JP)
(72) Inventor: YOSHIMI, Tatsunari, Tokyo 163-0410 (JP); KOJIMA, Satoshi, Tokyo 163-0410 (JP); AOYAGI, Katsumi, Tokyo 163-0410 (JP)
(74) Representative: Müller-Boré & Partner Patentanwälte PartG mbB
(86) International application number: PCT/JP2020/040896
(87) International publication number: WO 2021/085617

(57) **Abstract**

A method for measuring a steroid in a urine sample comprising:
an acid treatment step of mixing and reacting an urine sample with an acid solution such that a normality of acid becomes 0.6 to 4N to obtain an acid-treated solution;
a neutralization treatment step of mixing and reacting the acid-treated solution and a neutralizing solution to obtain a neutralization-treated solution; and
a measurement step of mixing the neutralization-treated solution and an antibody capable of specifically binding to a steroid to measure the steroid.

## Description

### [Technical Field]

The present invention relates to a method for measuring a steroid in a urine sample as well as a kit for measuring a steroid in a urine sample and a neutralizing solution for use in the method.

### [Background Art]

Measurements of steroids contained in blood samples and urine samples are used for diagnosis and differentiation of various diseases, and for example, measurement of aldosterone is useful for diagnosis and differentiation of hypertensive diseases including primary aldosteronism, kidney diseases, edematous diseases, and the like.

As a method for measuring a steroid in a sample, a method for immunologically measuring a steroid in a sample based on antigen-antibody reactions between the steroid and an antibody (anti-steroid antibody) for the steroid is known, and for example, as reagents for measuring aldosterone using anti-aldosterone antibodies, "LIAISON" (NPL 1) and "SPAC (registered trademark) -S aldosterone kit " (NPL 2) are sold by DiaSorin S.p.A and FUJIREBIO Inc., respectively.

In the urine excreted from the body, steroids normally bind to glucuronic acid and sulfuric acid to form conjugates. For this reason, if a urine sample is subjected to the immunological measurement based on antigen-antibody reaction as it is, the antigen-antibody reaction is hindered by other molecules that form conjugates to generate deviation between the actual amount of the steroid and the detected amount of the steroid, so that a correct measurement result cannot be obtained. Hence, a pretreatment to deconjugate the steroid from the conjugates is necessary. As the pretreatment, the conventional reagents for measuring aldosterone as described above require such treatment that hydrochloric acid of 0.2N is added to a urine sample to react for 16 to 24 hours, and thereafter, serum or a buffer is added for neutralization.

### [Citation List]

### [Non Patent Literature]

[NPL 1] "LIAISON" Package Insert, DiaSorin, revised 2014
[NPL 2] "SPAC (registered trademark) -S aldosterone kit" Package Insert, FUJIREBIO Inc., revised 2018

### [Summary of Invention]

### [Technical Problem]

Conventionally, the measurement of steroids in urine samples has often been conducted by an institute (testing company or the like) different from a facility (hospital or the like) where the samples have been collected from patients, and thus it has normally taken several days to obtain result of the measurement. However, in recent years, automation of immunological measurement devices has made it possible for a facility that has collected samples to conduct their own measurements of various biological substances, and the needs for measurements of steroids in urine samples have also been increasing. In the complete automation of measurement of steroids in urine samples, it is required that the operations from pretreatment of a sample (particularly deconjugation of a steroid) to the completion of the measurement can be consistently conducted on a measuring device. However, in the conventional methods, which take a long time (16 to 24 hours) to pretreat urine samples, the pretreatment is conducted outside the device. For this reason, a method for measuring a steroid that is capable of pretreating a urine sample in a shorter time and that can be completely automated including the pretreatment has been desired.

The present invention has been made in view of the above-described problems of the conventional techniques, and an object thereof is to provide a method for measuring a steroid in a urine sample as well as a kit for measuring a steroid in a urine sample and a neutralizing solution for use in the method that make it possible to pretreat a urine sample in a shorter time than the conventional techniques.

### [Solution to Problem]

The present inventors conducted earnest studies in order to achieve the above object, and consequently have found that surprisingly, it is possible to achieve sufficient effect of pretreatment similar to those of the conventional measuring methods (particularly, deconjugation of the steroid and the like) with acid treatment in a significantly shorter time than the conventional measuring methods by conducting acid treatment on a urine sample with a normality of acid made larger than the conventional measuring methods and within a specific range, and then neutralizing and using the sample after the pretreatment, and have led to the completion of the present invention.

Specifically, the present invention provides the following:
[1] A method for measuring a steroid in a urine sample, comprising:
   an acid treatment step of mixing and reacting an acid solution with a urine sample such that a normality of acid becomes 0.6 to 4N to obtain an acid-treated solution;
   a neutralization treatment step of mixing and reacting the acid-treated solution and a neutralizing solution to obtain a neutralization-treated solution; and
   a measurement step of mixing the neutralization-treated solution and an antibody capable of specifically binding to a steroid to measure the steroid.
[2] The method for measuring a steroid in a urine sample according to [1], wherein
   pH of the acid-treated solution at 20 to 40°C is 1.0 to 6.0.
[3] The method for measuring a steroid in a urine sample according to [1] or [2], wherein
   a reaction time in the acid treatment step is 1 to 60 minutes.
[4] The method for measuring a steroid in a urine sample according to any one of [1] to [3], wherein
   a normality of acid of the acid solution is 1 to 6N.
[5] The method for measuring a steroid in a urine sample according to any one of [1] to [4], wherein
   pH of the neutralization-treated solution at 25°C is 4.0 to 10.0.
[6] The method for measuring a steroid in a urine sample according to any one of [1] to [5], wherein
   the neutralizing solution contains at least one selected from the group consisting of Good's buffer agents, Tris buffer agents, sodium carbonate, sodium bicarbonate, sodium borate, and basic substances.
[7] The method for measuring a steroid in a urine sample according to any one of [1] to [6], wherein
   pKa of the neutralizing solution at 20°C is 6 to 8.
[8] A kit for measuring a steroid in a urine sample for use in the method for measuring a steroid in a urine sample according to any one of [1] to [7], comprising:
   at least one selected from the group consisting of
   an acid solution having a normality of acid of 1 to 6N,
   a neutralizing solution containing at least one selected from the group consisting of Good's buffer agents, Tris buffer agents, sodium carbonate, sodium bicarbonate, sodium borate, and basic substances, and
   an antibody capable of specifically binding to a steroid.
[9] A neutralizing solution for measuring a steroid in a urine sample for use in the method for measuring a steroid in a urine sample according to any one of [1] to [7], comprising:
   at least one selected from the group consisting of Good's buffer agents, Tris buffer agents, sodium carbonate, sodium bicarbonate, sodium borate, and basic substances.

### [Advantageous Effects of Invention]

The present invention makes it possible to provide a method for measuring a steroid in a urine sample as well as a kit for measuring a steroid in a urine sample and a neutralizing solution for use in the method that are capable of pretreating a urine sample in a shorter time than the conventional methods. In addition, this also makes it possible to consistently automate operations from pretreatment of a urine sample to completion of a measurement.

### [Brief Description of Drawings]

[FIG. 1] FIG. 1 is a graph showing relations between acid treatment time and ratios of measured value averages in Example 2-1 to Reference Example 2-1.
[FIG. 2] FIG. 2 is a graph showing relations between acid treatment time and a ratio of a measured value average in Example 2-2 to Reference Example 2-2.
[FIG. 3] FIG. 3 is a graph showing relations between acid treatment time and ratios of measured value averages in Example 4 to Reference Example 4.

### [Description of Embodiments]

### <Method for Measuring Steroid in Urine Sample>

The present invention provides a method for measuring a steroid in a urine sample, comprising:
an acid treatment step of mixing and reacting an acid solution with a urine sample such that a normality of acid becomes 0.6 to 4N to obtain an acid-treated solution;
a neutralization treatment step of mixing and reacting the acid-treated solution and a neutralizing solution to obtain a neutralization-treated solution; and
a measurement step of mixing the neutralization-treated solution and an antibody capable of specifically binding to a steroid to measure the steroid.

In the present invention, the "urine sample" is not particularly limited, and includes randomly collected urine, 24 hour-collected urine, and the like. In addition, the source of the urine sample is also not particularly limited, and includes, for example, human, as well as mammals and birds other than human (primates such as chimpanzee and monkey; and livestock and pet animals such as cattle, pig, horse, chicken, cat, and dog) . The urine sample according to the present invention may be diluted with a diluent as appropriate as long as the urine sample satisfies the normality of acid described below. The diluent includes, for example, at least one selected from the group consisting of buffer solutions, saline, purified water, serum, and plasma.

In the present invention, the "steroid" is a generic term of compounds having the steroid skeleton (cyclopentanoperhydrophenanthrene skeleton). The steroid includes steroid hormones and derivatives thereof that hold the steroid skeleton (example: synthetic steroids such as anabolic steroids, anti-androgen drugs, and anti-estrogen drugs). The steroid hormones include, for example, testicular hormones (example: testosterone), estrogens (example: estradiol), corpus luteum hormones (example: progesterone), and corticoids (example: glucocorticoids (cortisol and the like) and mineralocorticoids (aldosterone and the like)). The steroid to be applied to the present invention is not particularly limited, but is preferably at least one selected from the group consisting of aldosterone, progesterone, testosterone, cortisol, and estradiol, for example.

Aldosterone is one of steroid hormones (mineralocorticoids) secreted from the zona glomerulosa of the adrenal cortex, and promotes reabsorption of sodium ions, absorption of water content associated therewith, and efflux of hydrogen ions and potassium ions to be exchanged with sodium ions mainly in the renal tubes of the kidney.

Progesterone is one of steroid hormones (corpus luteum hormones) synthesized in the corpus luteum of the ovaries, and mainly has actions of changing the secretory phase of the endometrium, pregnancy maintenance, increasing the body temperature, ovulation inhibition, mammogenesis, and the like.

Testosterone is one of steroid hormones (androgens) secreted in the testicular interstitial cells, and mainly has action of promoting the growth of secondary sexual characteristics, action of increasing the muscle amount, action of forming the blood in the puberty of males.

Cortisol is one of steroid hormones (glucocorticoids) secreted from the adrenal cortex and mainly has action of adjusting carbohydrate·lipid·protein metabolism·nucleic acid metabolism.

Estradiol is one of steroid hormones (estrogens) produced in granulosa cells, theca externa cells, placenta, and adrenal cortex of the ovary and testicular interstitial cells, and mainly has actions such as growth stimulation of mammary cells, control on ovulation of the ovaries, control on lipid metabolism, insulin action, blood-clotting action, feminization of the central nerve (consciousness), thinning of skin, suppression of arteriosclerosis by decreasing LDL and increasing VLDL·HDL.

### [Acid Treatment Step]

In the method for measuring a steroid in a urine sample of the present invention, an acid solution is first mixed and reacted with a urine sample such that a normality of acid becomes 0.6 to 4N to obtain an acid-treated solution (acid treatment step).

In a urine sample, steroids normally bind to other molecules to form conjugates. For this reason, in this acid treatment step, the conjugated steroids are deconjugated to be free steroids. The other molecules that form conjugates with steroids include, for example, glucuronic acid; sulfuric acid; sugars such as N-acetyl glucosamine and glucose; and glycine. Note that in the present invention, the "conjugated" means that a steroid is bound to the other molecules, and the "deconjugated" means that a steroid is not bound to the other molecules and in a free state.

In the present invention, in the acid treatment step, it is necessary to mix an acid solution with the urine sample, and set a normality of acid in a solution after the mixing (preferably, a solution after the reaction, that is, after the elapse of the acid treatment time (in the Specification, referred to as an "acid-treated solution")) to 0.6 to 4N. Such a high acid concentration in the acid treatment step makes it possible to significantly shorten the time taken for deconjugation. The normality of acid can be adjusted as appropriate depending on the acid treatment time, the acid treatment temperature, and the type of the neutralizing solution described below, and the like, but is more preferably 1.3 to 3N, and particularly preferably 1.3 to 2N, from the viewpoint that there is a tendency that it becomes possible to make each operation efficient with more favorable acid treatment time and acid treatment temperature. If the normality of acid is less than the lower limit, the acid treatment tends to take a longer time. On the other hand, if the normality of acid is more than the upper limit, there is a tendency that the time of the acid treatment becomes too short, so that it becomes impossible to secure time for preparation of the next operation, or neutralization becomes difficult, so that the measured value decreases. Note that in the present invention, the "normality of acid" in the solution can be measured by neutralization titration of an aqueous solution of sodium hydroxide.

In addition, the pH of the solution (preferably, an acid-treated solution) after the mixing of the urine sample and the acid solution at 20 to 40°C (preferably, 25°C) is preferably 1.0 to 6.0, and more preferably 1.0 to 4.9, from the same viewpoint as described above.

The acid solution to be mixed with the urine sample includes an aqueous solution that exhibits acidic property. The acid solution according to the present invention includes hydrochloric acid, sulfuric acid, and p-toluenesulfonic acid, and one of these may be used alone or a mixture of two or more of these may be used. In addition, the acid solution according to the present invention may further contain methanol or the like as long as the effect of the present invention is not hindered.

In the acid solution according to the present invention, the concentration of acid is not particularly limited as long as the concentration makes it possible to set the normality of acid in the solution after the acid solution is mixed with the urine sample within the above range. For example, if the acid solution is intended to be diluted n-fold when mixed with the urine sample, an acid solution containing the acid of n-fold concentration may be prepared in advance. From the viewpoint of the safety and efficiency of the operation, as the concentration of acid in the acid solution according to the present invention, the normality of acid is preferably 1 to 6N, more preferably 2 to 4N, and further preferably 2 to 3N.

In the acid treatment step according to the present invention, the mixing ratio of the acid solution to the urine sample is not particularly limited as long as the mixing ratio makes it possible to set the normality of acid in the solution after the mixing within the above range, but is preferably a mixing ratio of 10 to 1000 µL of the acid solution to 100 µL of the urine sample from the viewpoint of the work efficiency, for example.

According to the method for measuring a steroid in a urine sample of the present invention, it is possible to shorten the reaction time (acid treatment time) sufficiently in the acid treatment step, and specifically shorten the reaction time to 1 to 60 minutes. The reaction time can be adjusted as appropriate depending on the normality of acid in the acid solution, the acid treatment temperature, and the type of the neutralizing solution described below, and the like, but is preferably 1 to 10 minutes, more preferably 1 to 7 minutes, further preferably 5 to 7 minutes, and even more preferably 5 to 6 minutes, from the viewpoint that there is a tendency that it becomes possible to make each operation efficient with more favorable acid concentration and acid treatment temperature. If the acid treatment time is less than the lower limit, there is a tendency that the deconjugation of the steroid becomes insufficient, so that the measured value decreases. On the other hand, if the acid treatment time is more than the upper limit, there is a tendency that the steroid itself is decomposed or altered by excessive acid hydrolysis, so that the measured value decreases.

In the acid treatment step according to the present invention, the reaction temperature (acid treatment temperature) is not particularly limited, and may be, for example, within a range of 5 to 95°C. The reaction temperature can be adjusted as appropriate depending on the normality of acid in the acid solution, the acid treatment time, and the type of the neutralizing solution, and the like, but is preferably 20 to 40°C from the viewpoint that there is a tendency that it becomes possible to make each operation efficient with more favorable acid concentration and acid treatment time. If the acid treatment temperature is less than the lower limit, there is a tendency that the deconjugation of the steroid becomes insufficient, so that the measured value decreases. On the other hand, if the acid treatment temperature is more than the upper limit, there is a tendency that the steroid itself is decomposed or altered by excessive acid hydrolysis, so that the measured value decreases.

Examples of such acid treatment conditions include, for example, conditions in which the normality of acid in the acid-treated solution is 0.6 to 4N, the acid treatment time is 1 to 60 minutes, and the acid treatment temperature is 5 to 95°C; conditions in which the normality of acid in the acid-treated solution is 1.3 to 3N, the acid treatment time is 1 to 10 minutes (more preferably 1 to 7 minutes), and the acid treatment temperature is 20 to 40°C; conditions in which the normality of acid in the acid-treated solution is 1.3 to 2N, the acid treatment time is 1 to 10 minutes (more preferably 1 to 7 minutes), and the acid treatment temperature is 20 to 40°C; and conditions in which the normality of acid in the acid-treated solution is 1.3 to 2N, the acid treatment time is 5 to 6 minutes, and the acid treatment temperature is 20 to 40°C.

### [Neutralization Treatment Step]

In the method for measuring a steroid in a urine sample of the present invention, subsequently, a solution (acid-treated solution) after the urine sample and the acid solution are mixed and reacted in the acid treatment step and a neutralizing solution are mixed and reacted to obtain a neutralization-treated solution (neutralization treatment step). In this neutralization treatment step, mainly in order to suppress denaturation of the antibody and the like to be used in the measurement step described below, the acid added in the acid treatment step is neutralized.

In the neutralization treatment step, the pH of the solution after the mixing of the acid-treated solution and the neutralizing solution (preferably, a solution after the reaction, that is, after the elapse of the neutralization treatment time (in the Specification, referred to as a "neutralization-treated solution")) at 25°C is preferably 4.0 to 10.0, and more preferably 5.0 to 8.0. If the pH is out of the above range, there is a tendency that the antibody and the like to be used in the measurement step described below is denaturated, so that the measured value decreases.

The neutralizing solution according to the present invention includes, for example, an aqueous solution that is based on a buffer solution (buffer) and may contain an additive as necessary as long as the buffer solution capacity is not hindered. The buffer solution is preferably at least one selected from the group consisting of Good's buffer solutions (HEPES buffer, MOPS buffer, and the like), Tris buffer solutions (Tris-HCl buffer solution, TE buffer solution, TAE buffer solution, TBE buffer solution, Tris-buffered saline, and the like), carbonate-bicarbonate buffer solution, and sodium borate buffer solution.

In addition, the neutralizing solution according to the present invention also includes, for example, an aqueous solution of a basic substance that may contain an additive as necessary. The basic substance includes, for example, hydroxides of alkali metals (NaOH, KOH, Ca(OH)₂, and the like), ammonia, and amines.

Moreover, the neutralizing solution according to the present invention also includes, for example, a solution that is based on serum and may contain an additive as necessary as long as the solution contains no steroids. The serum is not particularly limited, but includes, for example, serums derived from animals (serums derived from animals such as human, cattle, horse, sheep, goat, pig, llama, dog, chicken, donkey, cat, rabbit, guinea pig, hamster, rat, mouse, and the like), and delipidized serum (DDC Mass Spect Gold (manufactured by VERITAS Corporation) and the like).

The neutralizing solution according to the present invention is not particularly limited as long as the neutralizing solution has a neutralizing capacity of neutralizing the acid in the acid-treated solution, but among those above, the neutralizing solution is preferably at least one selected from the group consisting of aqueous solutions based on various buffer solutions, aqueous solutions of basic substances, and delipidized serum, and more preferably an aqueous solution based on any of various buffer solutions and/or an aqueous solution of a basic substance from a viewpoint that variations among lots due to a raw material can be reduced and from the viewpoint that there is a tendency that it becomes possible to make each operation efficient with more favorable acid concentration and acid treatment time, and further preferably an aqueous solution based on any of the above buffer solutions from the viewpoint that generation of salts and the like can be suppressed.

More specifically, the aqueous solution based on any of the above various buffer solutions and an aqueous solution of a basic substance include an aqueous solution containing at least one selected from the group consisting of Good's buffer agents, Tris buffer agents, sodium carbonate, sodium bicarbonate, sodium borate, and basic substances.

The Good's buffer agents include, for example, MES, Bis-Tris, ADA, PIPES, ACES, MOPSO, BES, MOPS, TES, HEPES, TAPSO, POPSO, HEPSO, EPPS, tricine, bicine, TAPS, CHES, and CAPS. In addition, the Tris buffer agents include, for example, Tris, Tris-HCl, and Tris-maleate.

In addition, more specifically, it is preferable that the neutralizing solution according to the present invention have pKa of 6 to 8 at 20°C. If the pKa is within the above range, it is possible to sufficiently neutralize the acid-treated solution to increase pH thereof and to thus suppress the denaturation of the antibody and the like to be used in the measurement step described below, which makes it possible to increase the normality of acid in the acid treatment step to further shorten the acid treatment time.

The neutralizing solution according to the present invention may further contain one or a combination of two or more of molecules for adjusting salt concentration such as sodium chloride, preservatives (antiseptics) such as sodium azide, protein components such as bovine serum albumin (BSA), blocking agents, protein stabilizers, sensitizers, sugars, hydrophilic polymers (for example, polyethylene glycol and polyvinyl alcohol), and the like as the additive as long as the effect of the present invention is not hindered.

In addition, it is also preferable that the neutralizing solution according to the present invention further include cyclodextrin from the viewpoint that the steroid is expected to be stabilized. In the present invention, the "cyclodextrin" represents a generic term of cyclic oligosaccharides having cyclic structures in which a plurality of glucoses are bound, includes, for example, α-cyclodextrin, β-cyclodextrin, hydroxypropyl-p-cyclodextrin, and γ-cyclodextrin, and may be one of these or two or more of these in combination.

In the neutralization treatment step according to the present invention, the mixing ratio between the acid-treated solution and the neutralizing solution is not particularly limited as long as neutralization is possible (preferably, as long as the pH of the neutralization-treated solution can be within the above range). However, the mixing ratio may be a mixing ratio of 10 to 1000 µL of the neutralizing solution to 100 µL of the acid-treated solution from the viewpoint of work efficiency, for example.

In the neutralization treatment step according to the present invention, the time for neutralization treatment (neutralization treatment time) is not particularly limited, but is normally 1 to 10 minutes. In addition, in the neutralization treatment step according to the present invention, the temperature (neutralization treatment temperature) is also not particularly limited, and is, for example, within a range of 5 to 95°C, and is preferably 20 to 40°C from the viewpoint that setting the same temperature as in the acid treatment step is simple and easy.

### [Measurement Step]

In method for measuring a steroid in a urine sample of the present invention, subsequently, the neutralization-treated solution after the neutralization treatment step and an antibody capable of specifically binding to a steroid are mixed to measure the steroid (measurement step).

In the case where an antibody is added to conduct antigen-antibody reaction while the steroid in the urine sample has formed conjugates, the antigen-antibody reaction is hindered to generate deviation between the actual amount of the steroid and the amount of the steroid to be detected, so that the measured value decreases. In contrast, in the method for measuring a steroid in a urine sample according to the present invention, since the pretreatments in the acid treatment step and the neutralization treatment step sufficiently deconjugate the steroid, it is possible to suppress occurrence of such deviation.

In the present invention, the "measurement" includes detection to take out the presence or absence and the amount of the steroid in the sample (neutralization-treated solution) as signals, and also the quantification or semi-quantification of the amount of the steroid. In the present invention, the measurement of the steroid is preferably conducted by detecting a signal generated by a labeling substance and quantifying this signal as necessary. The "signal" includes color reaction (color production), reflected light, luminescence, fluorescence, radiation by radioisotope, and the like, and also includes what can be observed by a measuring method·device depending on the type of the signal besides what can be observed by the naked eye.

In the measurement step according to the present invention, the immunological measurement by means of antigen-antibody reaction is conducted using an antibody capable of specifically binding to a steroid. Such a measuring method includes, for example, immunological approaches such as the CLEIA (chemiluminescent enzyme immunoassay) and the EIA (enzyme immunoassay) using enzymes as labels (labeling substances); the RIA (radioimmunoassay) using radioisotopes as labels; the CLIA (chemiluminescent immunoassay) using chemiluminescent compounds as labels; the latex agglutination method; and the immunochromatography, but is not limited to these. In addition, each of these immunological approaches may be a non-competitive assay or may be a competitive assay.

In the present invention, the "antibody capable of specifically binding to a steroid (hereinafter sometimes referred to as an "anti-steroid antibody") " is not particularly limited as long as the antibody is capable of binding to a steroid, and may be a polyclonal antibody or may be a monoclonal antibody (chimeric antibody, humanized antibody, human antibody, and the like). In addition, the antibody may be any isotype such as IgG, IgM, IgA, IgD, IgE, or IgY. In the present invention, the "antibody" includes not only complete antibodies but also antibody fragments (for example, Fab, Fab', F(ab')₂, Fv, single-chain antibodies, diabodies, and the like) and low molecular weight antibodies in which a variable region of an antibody is bound. The anti-steroid antibody according to the present invention is preferably a monoclonal antibody. As the monoclonal antibody, for example, one prepared by a publicly-known method such as the hybridoma method or the recombinant DNA method may be used.

As the anti-steroid antibody according to the present invention, an antibody that is bound with a labeling substance can be used. The labeling substance is not particularly limited as long as the labeling substance can be bound to an antibody and be detected, and includes, for example, enzymes such as alkaline phosphatase (ALP), horseradish peroxidase (HRP), and β galactosidase (β-gal); fluorescent dyes such as fluorescein isothiocyanate (FITC) and rhodamine isothiocyanate (RITC); radioisotopes such as ¹²⁵I; fluorescent protein such as allophycocyanin (APC) and phycoerythrin (R-PE); avidin; biotin; latex; and gold particles, but is not limited to these.

In the case where an enzyme is used as the labeling substance, it is possible to detect various signals depending on a substrate by using, for example, a chromogenic substrate, a fluorescent substrate, a chemiluminescent substrate, or the like as the substrate.

As a method for measuring a steroid in the present invention, besides a method for directly detecting a steroid in a sample (neutralization-treated solution) using an anti-steroid antibody bound with the labeling substance, a method for indirectly detecting a steroid using a secondary antibody or the like bound with the labeling substance without binding the labeling substance to the anti-steroid antibody may also be used. Here, the "secondary antibody" is an antibody exhibiting reactivity against an anti-steroid antibody. For example, in the case where a mouse antibody is prepared as the anti-steroid antibody, an anti-mouse IgG antibody can be used as the secondary antibody. As the secondary antibody, labeled secondary antibodies that can be used respectively for antibodies derived from various biological species such as rabbit, goat, and mouse are commercially available, and an appropriate secondary antibody can be selected and used depending on a biological species from which the anti-steroid antibody is derived. In addition, protein G, protein A, or the like bound with the labeling substance can also be used instead of the secondary antibody.

For binding the anti-steroid antibody and the labeling substance, or the secondary antibody and the labeling substance, a conventionally known method can be employed as appropriate. In addition, a biotin-avidin system can also be used for this binding. In this method, for example, an anti-steroid antibody is biotinylated, and the biotinylated anti-steroid antibody is interacted with an avidinylated labeling substance to bind the labeling substance to the anti-steroid antibody utilizing interaction between biotin and avidin.

As the principle for detection in the above immunological approach, the sandwich method and the competitive method are favorable. In the sandwich method, a substance to be detected (for example, an antigen (steroid)) is captured by a capturing antibody (for example, an anti-steroid antibody) fixed (immobilized) to a solid phase, the capturing antibody is then recognized by a detecting antibody bound with a labeling substance (for example, an anti-steroid antibody bound with a labeling substance, a secondary antibody bound with a labeling substance, or the like) to detect the substance depending on the type of the labeling substance. As the solid phase, for example, fine beads such as magnetic beads and latex beads, a plate such as a plastic plate, or a fibrous substance of nitrocellulose or the like can be used.

The capturing antibody may be fixed directly to the solid phase, but may be fixed indirectly. For example, it is possible to fix a substance that binds to the capturing antibody to the solid phase, and binds the capturing antibody to the substance to fix the capturing antibody indirectly to the solid phase. The substance that binds to the capturing antibody includes, for example, secondary antibodies, protein G, protein A, and the like, but is not limited to these. For the fixation to the solid phase, a conventionally known method can be employed as appropriate. In addition, in the case where the capturing antibody is biotinylated, an avidinylated solid phase can be used.

In the sandwich method, for example, it is preferable to mix the neutralization-treated solution and the immobilized capturing antibody to conduct a first antigen-antibody reaction (first reaction), forming a complex (immune complex) of the steroid in the neutralization-treated solution and the capturing antibody, and after separating and washing this immune complex as necessary, to add a detecting antibody bound with the labeling substance to this to conduct a second antigen-antibody reaction (second reaction) and conduct various detection depending on the labeling substance.

As the detecting antibody, it is possible to use not only an antibody that binds only to a steroid but also an antibody that binds specifically to a complex of a steroid and an antibody.

As the sandwich method, for example, the sandwich CLIA, which is one mode of the CLIA, the sandwich CLEIA, which is one mode of the CLEIA, and the IRMA (immunoradiometric assay), which is one mode of the RIA, are favorable.

On the other hand, as the competitive method, for example, in a first mode, a substance to be detected (steroid) in a sample and an immobilized competitive substance (the same steroid as the substance to be detected or a substance that cross-reacts with this) are caused to compete for binding to a detecting antibody bound with a labeling substance (for example, an anti-steroid antibody bound with the labeling substance). For example, it is preferable to conduct antigen-antibody reaction by mixing the neutralization-treated solution, the detecting antibody bound with the labeling substance, and the immobilized competitive substance.

In addition, for example, in a second mode of the competitive method, the substance to be detected in the sample and the competitive substance bound with a labeling substance are caused to compete for binding to the immobilized capturing antibody. For example, it is preferable to conduct antigen-antibody reaction by mixing the neutralization-treated solution and the competitive substance bound with the labeling substance, and adding the immobilized capturing antibody to the mixture.

For the fixation of the competitive substance and the capturing antibody to the solid phase in the competitive method, a conventionally known method can be employed as appropriate. Such fixation is the same as the fixation of the capturing antibody to the solid phase in the above sandwich method, and may be direct or indirect.

In the measurement step according to the present invention, the neutralization-treated solution can be subjected to the measurement as it is. However, in the case where cyclodextrin is added to the neutralizing solution, or the like, for example, a treatment solution containing a surfactant may be added to a neutralization-treated solution and/or an antigen-antibody reaction system before the measurement step. The treatment solution containing a surfactant includes, for example, one described in International Publication No. WO2019/098314. As the treatment solution containing a surfactant, for example, the treatment solution may be added in advance to another solution used in the antigen-antibody reaction system (for example, the detecting antibody bound with the labeling substance in the first mode of the competitive method, the competitive substance bound with the labeling substance in the second mode of the competitive method, or the like) to be used, or the surfactant may be added in advance to these solutions to serve as the treatment solution.

The quantification of the amount of the steroid from the measured value obtained in the measurement step according to the present invention is generally conducted by comparing the measured value with measured values obtained from standard solutions containing the steroid of the respective concentrations. In this case, for example, the amount of the steroid in the urine sample can be obtained by checking at which position the measured value obtained in the test sample (neutralization-treated solution) is located on a standard curve created based on measured values from the standard solutions.

Since the method for measuring a steroid in a urine sample of the present invention can measure the concentration of a steroid based on which diseases related to abnormality of the amount of the steroid hormone secreted are diagnosed (evaluation of the contraction or the risk), the method can be used for the diagnosis. Here, the "diseases related to abnormality of the amount of the steroid hormone secreted" mean both diseases attributable to abnormality of the amount of the steroid hormone secreted and diseases which make the amount of the steroid hormone secreted abnormal as a result of the onsets of such diseases.

For example, it is known that in primary aldosteronism, renovascular hypertension, malignant hypertension, renin-producing tumors, Bartter's syndrome, edematous diseases (liver cirrhosis · heart failure), and the like, the concentration of aldosterone in the blood exhibits high values, while in Addison's disease, 21-hydroxylase deficiency, selective hypoaldosteronism, and the like, the concentration of aldosterone exhibits low values. In addition, it is known that in congenital adrenal hyperplasia, Cushing's syndrome, adrenal tumors, and the like, the concentration of progesterone in the blood exhibits high values, while in ovarian failure, luteal phase deficiency, and the like, the concentration of progesterone exhibits low values. Moreover, it is known that in liver diseases, estrogen-producing tumors, congenital adrenal hyperplasia, multiple pregnancy, ovarian hyperstimulation syndrome, and the like, the concentration of estradiol in the blood exhibits high values, while in ovarian failure, premature ovarian failure, hypogonadotropic disease, Chiari-Frommel syndrome, and the like, the concentration of estradiol exhibits low values.

### <Kit for Measuring a Steroid in a Urine Sample and Neutralizing Solution>

The present invention also provides a kit for measuring a steroid in a urine sample and a neutralizing solution for use in the above method for measuring a steroid in a urine sample of the present invention.

A kit for measuring a steroid in a urine sample of the present invention comprises:
at least one selected from the group consisting of
an acid solution having a normality of acid of 1 to 6N,
a neutralizing solution containing at least one selected from the group consisting of Good's buffer agents, Tris buffer agents, sodium carbonate, sodium bicarbonate, sodium borate, and basic substances, and
an antibody capable of specifically binding to a steroid. In addition, a neutralizing solution for measuring a steroid in a urine sample of the present invention comprises:
   at least one selected from the group consisting of Good's buffer agents, Tris buffer agents, sodium carbonate, sodium bicarbonate, sodium borate, and basic substances.

The acid solution, the neutralizing solution, and the antibody capable of specifically binding to a steroid (anti-steroid antibody) are as described above including their preferable modes.

The kit of the present invention may be further combined with, for example, a standard steroid reagent, a treatment solution containing a surfactant, a control reagent, a cleaning liquid, a diluent, a dilution cartridge, and the like. In addition, for example, in the case where an enzyme label is used as the labeling substance, a substrate, a reaction stop solution, and the like necessary for detecting the label may be included in the kit.

Moreover, in the case where the sandwich method is employed as the detection principle, for example, an immobilized steroid-capturing antibody, a solid phase to which a substance that binds to a steroid-capturing antibody is fixed, a detecting antibody bound with a labeling substance, and the like may be included in the kit. On the other hand, in the case where the competitive method is employed as the detection principle, for example, besides these, an immobilized competitive substance, a solid phase to which a substance that binds to a competitive substance is fixed, and the like may be included in the kit.

In addition, in the case where the primary antibody (antibody that directly binds to an antigen) or the secondary antibody is not labeled, for example, labeled substances that bind to these antibodies may be included in the kit. In addition, in the case where the anti-steroid antibody is biotinylated, for example, an avidinylated labeling substance may be included in the kit. an instruction manual of the kit may be further included in the kit of the present invention.

The kit and the neutralizing solution of the present invention may be used not only for research but also, as a pharmaceutical product for in-vitro diagnosis, for the quantification of the concentration of the steroid based on which the above diseases related to abnormality of the amount of the steroid hormone secreted are diagnosed, for example.

### [Examples]

Hereinafter, the present invention will be described more specifically based on Examples, Reference Examples, and Comparative Examples; however the present invention is not limited to Examples below. Note that hereinafter, the indication of "%" represents weight/volume (w/v) percent (g/100mL) unless otherwise stated.

### <Shortening of Time for Pretreatment of Urine Sample>

### (Reference Example 1-1)

### [Pretreatment]

As urine samples, a randomly collected urine sample (sample A) of volunteer A, a randomly collected urine sample (sample B) of volunteer B, and a randomly collected urine sample (sample C) of volunteer C were used. Each urine sample and hydrochloric acid of 0.2N were mixed such that the volume ratio (urine sample:hydrochloric acid) became 1:2, followed by incubating at 30°C for 18 hours to conduct acid treatment. Subsequently, a neutralizing solution (50 mM MOPS, 150 mM NaCl, 1% HPβCD (2-Hydroxypropyl-β-Cyclodextrin), 0.025% Lipidure 802 (manufactured by NOF Corporation), pH 7.5, pKa (pKa at 20°C, the same applies hereinafter)=7.2) having MOPS concentration of 50 mM was mixed with the solution (acid-treated solution, 0.13N hydrochloric acid) after the acid treatment such that the volume ratio (acid-treated solution:neutralizing solution) became 1:9 to prepare a measurement sample (neutralization-treated solution).

### [Measurement of Steroid]

The measurement was conducted by a two-step method using an anti-aldosterone antibody-bound beads (anti-aldosterone antibody-bound magnetic beads prepared by a conventional method (antibody-bound beads)) and an alkaline phosphatase-labeled anti-aldosterone immune complex antibody (alkaline phosphatase-labeled anti-aldosterone antibody prepared by a conventional method (enzyme-labeled antibody)) for each measurement sample. Specifically, the measurement was conducted in accordance with steps (a) to (g) as follows:
(a) About 30 µL of the measurement sample is added to about 50 µL of the antibody-bound beads, followed by agitating and reacting at 37°C for 8 minutes (first reaction).
(b) The magnetic beads are magnetically collected using a magnetic separator, followed by washing.
(c) About 50 µL of the enzyme-labeled antibody is added, followed by agitating and reacting at 37°C for 8 minutes (second reaction).
(d) The magnetic beads are magnetically collected using a magnetic separator, followed by washing.
(e) about 200 µL of AMPPD (substrate, 3-(2'-spiroadamane)-4-methoxy-4-(3"-phosphoryloxy)phe nyl-1,2-dioxetane·2 sodium salt) is added, followed by agitating and reacting at 37°C for 4 minutes (enzyme reaction).
(f) The amount of luminescence (count) of light having an emission maximum peak near a wavelength of 463 nm, which is emitted along with the decomposition of the substrate is measured. As the result of measurement of the amount of luminescence, an average value (count average) of quadruple measurements (n=4) is taken.
(g) Steps (a) to (f) were conducted using a standard aldosterone reagent (manufactured by FUJIREBIO Inc.) instead of the measurement samples to create a standard curve. The amount of aldosterone in the measurement sample is calculated using the created standard curve from the count measured in (f) as a measured value (pg/mL) . As the result, an average (measured value average) of each measured value calculated from each count value of quadruple measurements (n=4) is taken.

### (Reference Example 1-2)

A measurement sample was prepared by changing the neutralizing solution to a neutralizing solution having a MOPS concentration of 1M (1M MOPS, 150 mM NaCl, 1% HPβCD, 0.025% Lipidure 802 (manufactured by NOF Corporation) , pH 7.5, pKa=7.2). The steroid measurement was conducted in the same manner as in Reference Example 1-1 except that the measurement sample obtained was used.

### (Comparative Example 1)

A measurement sample was prepared by changing the incubation conditions after each urine sample (samples A to C) and 0. 2N hydrochloric acid were mixed to at 37°C for 6.5 minutes, and changing the neutralizing solution to a neutralizing solution having a MOPS concentration of 1M (1M MOPS, 150 mM NaCl, 1% HPβCD, 0.025% Lipidure 802 (manufactured by NOF Corporation) , pH 7.5, pKa=7.2). The steroid measurement was conducted in the same manner as in Reference Example 1-1 except that the measurement sample obtained was used.

### (Example 1)

Each of the urine samples (samples A to C) and hydrochloric acid of 1N (Example 1-1), 2N (Example 1-2), 3N (Example 1-3), 4N (Example 1-4), or 5N (Example 1-5) were mixed such that the volume ratio (urine sample:hydrochloric acid) became 1:2, followed by incubation at 37°C for 6.5 minutes to conduct acid treatment. Subsequently, a neutralizing solution having a MOPS concentration of 1M (1MMOPS, 150 mM NaCl, 1% HPβCD, 0.025% Lipidure 802 (manufactured by NOF Corporation) , pH 7.5, pKa=7.2) was mixed with the solution after the acid treatment (acid-treated solution, 0.67N hydrochloric acid (Example 1-1), 1.33N hydrochloric acid (Example 1-2), 2N hydrochloric acid (Example 1-3), 2.67N hydrochloric acid (Example 1-4), or 3.33N hydrochloric acid (Example 1-5)) such that the volume ratio (acid-treated solution:neutralizing solution) became 1:9 to obtain a measurement sample (neutralization-treated solution). The steroid measurement was conducted in the same manner as in Reference Example 1-1 except that the measurement sample obtained was used.

The results of count averages and measured value averages of samples A to C in Reference Examples 1-1 to 1-2, Comparative Example 1, and Examples 1-1 to 1-5 are shown in Table 1 below. In addition, Table 1 also shows the ratios of the count average and the measured value average (count vs control (%), measured value vs control (%)) in each of Reference Example 1-2, Comparative Example 1 and each Example, where the count average and the measured value average in Reference Example 1-1 were each regarded as 100% (control). Although the ratio of the measured value average to the control may be different depending on the measurement system, it can be determined that the ratio of 25% or more means it has sufficient deconjugation capacity that is usable in steroid measurement (the same applies hereinafter).

**[Table 1]**

| | | Reference Example 1-1 (Control) | Reference Example 1-2 | Comparative Example 1 | Example 1-1 | Example 1-2 | Example 1-3 | Example 1-4 | Example 1-5 |
|---|---|---|---|---|---|---|---|---|---|
| Concentration of HCl | | 0.2N | 0.2N | 0.2N | IN | 2N | 3N | 4N | 5N |
| Acid treatment conditions | | 30°C 18h | 30°C 18h | 37°C 6.5min | | | | | |
| MOPS concentration of neutralizing solution | | 50mM | 1M | | | | | | |
| Count average (n=4) | Sample A | 48051 | 52347 | 4465 | 19340 | 45138 | 47121 | 29518 | 10117 |
| | Sample B | 1187069 | 1205090 | 46955 | 503714 | 1099527 | 1124734 | 795903 | 255582 |
| | Sample C | 2021517 | 2076350 | 107129 | 1079628 | 1891440 | 1962788 | 1596823 | 739614 |
| Count vs control | Sample A | 100% | 109% | 9% | 40% | 94% | 98% | 61% | 21% |
| | Sample B | 100% | 102% | 4% | 42% | 93% | 95% | 67% | 22% |
| | Sample C | 100% | 103% | 5% | 53% | 94% | 97% | 79% | 37% |
| Measured value average (pg/mL) (n=4) | Sample A | 92.7 | 97.5 | 11 | 51.3 | 89.2 | 91.6 | 68.4 | 30.6 |
| | Sample B | 757.9 | 769.3 | 91.4 | 373.1 | 703.1 | 718.7 | 526.6 | 243.7 |
| | Sample C | 1394.2 | 1444.5 | 147.6 | 691 | 1279 | 1341.3 | 1041.4 | 495.9 |
| Measure value vs control | Sample A | 100% | 105% | 12% | 55% | 96% | 99% | 74% | 33% |
| | Sample B | 100% | 102% | 12% | 49% | 93% | 95% | 69% | 32% |
| | Sample C | 100% | 104% | 11% | 50% | 92% | 96% | 75% | 36% |

As shown in Table 1, it was confirmed that in the case where the normality of acid in the acid-treated solution was set to 0.6N or more (for example, Examples 1-1 to 1-5), even when the time for acid treatment is shortened to 6.5 minutes, measurement results similar to those in the case where the acid treatment was conducted under the conventional long-time condition (for example, Reference Examples 1-1 to 1-2) were obtained and the steroid was sufficiently deconjugated. On the other hand, it was suggested that in the case where hydrochloric acid of 0.2N was used as in the conventional techniques, that is, in the case where the normality of acid in the acid-treated solution was around 0.13N (for example, Comparative Example 1), when the time for acid treatment was shortened to 6.5 minutes, the count value average and the measured value average significantly decreased and the steroid was not sufficiently deconjugated even with the same sample.

### <Acid Treatment Time in Pretreatment of Urine Sample, Comparison 1 of Neutralizing Solution>

### [Buffer Neutralizing Solution]

### (Reference Example 2-1)

A randomly collected urine sample of volunteer D was used as a urine sample (sample D). The urine sample and hydrochloric acid of 0.2N were mixed such that the volume ratio (urine sample: hydrochloric acid) became 1:2, followed by incubation at 30°C for 18 hours to conduct acid treatment. Subsequently, a buffer having a MOPS concentration of 2M (2M MOPS, 150 mM NaCl, 1% HPβCD, 0.025% Lipidure 802 (manufactured by NOF Corporation), pH 7.3, pKa=7.2) was mixed as a neutralizing solution with the solution after the acid treatment (acid-treated solution, 0.13N hydrochloric acid) such that the volume ratio (acid-treated solution:neutralizing solution) became 1:9 to obtain a measurement sample (neutralization-treated solution) . The steroid measurement was conducted in the same manner as in Reference Example 1-1 except that the measurement sample obtained was used.

### (Example 2-1)

The urine sample (sample D) and hydrochloric acid of 3N, 4N, 5N, or 6N were mixed such that the volume ratio (urine sample:hydrochloric acid) became 1:2, followed by incubation at 37°C for 1, 2, 3, 4, 5, or 6 minutes to conduct acid treatment. Subsequently, a buffer having a MOPS concentration of 2M (2M MOPS, 150 mM NaCl, 1% HPβCD, 0.025% Lipidure 802 (manufactured by NOF Corporation), pH 7.3, pKa=7.2) was mixed as a neutralizing solution with the solution after the acid treatment (acid-treated solution, hydrochloric acid of 2N, 2.67N, 3.33N, or 4N) such that the volume ratio (acid-treated solution:neutralizing solution) became 1:9 to obtain a measurement sample (neutralization-treated solution). The steroid measurement was conducted in the same manner as in Reference Example 1-1 except that the measurement sample obtained was used.

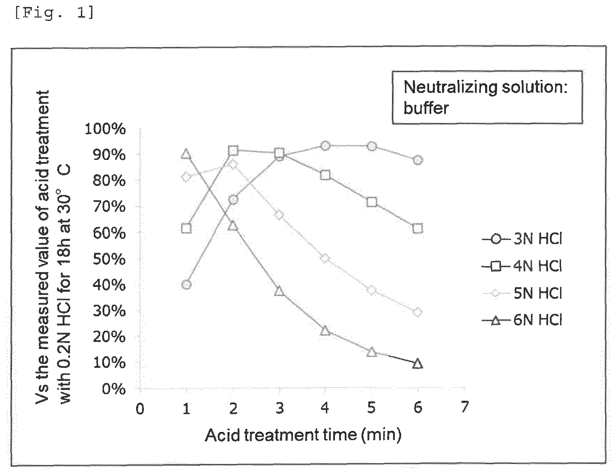

FIG. 1 shows relations between the acid treatment time and the ratio of each of the measured value averages in Example 2-1 (vs the measured value of acid treatment with 0.2N HCl for 18h at 30°C (%)) where the measured value average in Reference Example 2-1 was regarded as 100% (control).

### [Delipidized Serum Neutralizing Solution]

### (Reference Example 2-2)

The urine sample (sample D) and hydrochloric acid of 0.2N were mixed such that the volume ratio (urine sample:hydrochloric acid) became 1:2, followed by incubation at 30°C for 18 hours to conduct acid treatment. Subsequently, delipidized serum ("DDC Mass Spect Gold", steroid hormone and cholesterol·TG-free serum, manufactured by VERITAS Corporation) was mixed as a neutralizing solution with the solution after the acid treatment (acid-treated solution, 0.13N hydrochloric acid) such that the volume ratio (acid-treated solution:neutralizing solution) became 1:9 to obtain a measurement sample (neutralization-treated solution). The steroid measurement was conducted in the same manner as in Reference Example 1-1 except that the measurement sample obtained was used.

### (Example 2-2)

The urine sample (sample D) and hydrochloric acid of 3N were mixed such that the volume ratio (urine sample:hydrochloric acid) became 1:2, followed by incubation at 37°C for 1, 2, 3, 4, 5, or 6 minutes to conduct acid treatment. Subsequently, delipidized serum ("DDC Mass Spect Gold" , steroid hormone and cholesterol ·TG-free serum, manufactured by VERITAS Corporation) was mixed as a neutralizing solution with the solution after the acid treatment (acid-treated solution, 2N hydrochloric acid) such that the volume ratio (acid-treated solution:neutralizing solution) became 1:9 to obtain a measurement sample (neutralization-treated solution) . The steroid measurement was conducted in the same manner as in Reference Example 1-1 except that the measurement sample obtained was used.

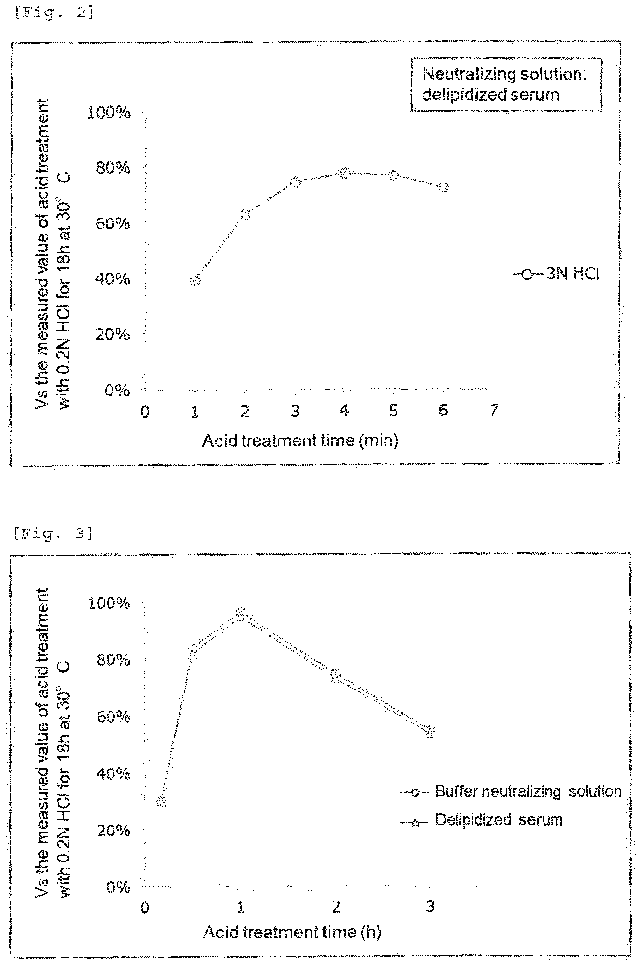

FIG. 2 shows a relation between the acid treatment time and the ratio of the measured value average in Example 2-2 (Vs the measured value of acid treatment with 0.2N HCl for 18h at 30°C (%)) where the measured value average in Reference Example 2-2 was regarded as 100% (control).

As shown in FIG. 1, it was confirmed that there was a tendency that the larger the normality of acid in acid treatment was, the shorter the time for the acid treatment became. Moreover, as shown in FIG. 2, it was confirmed that even when delipidized serum was used as a neutralizing solution (for example, Example 2-2), it was possible by acid treatment for a short time to obtain a measurement result at a level near that in the case where acid treatment was conducted under the conventional long-time condition (for example, Reference Example 2-2) . However, in the case where a neutralizing solution based on delipidized serum was used, there was a tendency that when the normality of acid in acid treatment was further increased, the value of the measured value average decreased. This is surmised to be because since the neutralizing capacity of delipidized serum is lower than buffer solutions, the acid-treated solution having a high acid concentration was not sufficiently naturalized, and the antigen-antibody reaction was hindered. From these results, it was confirmed that since it was possible to make the acid concentration in acid treatment higher in the case where a neutralizing solution based on a buffer solution was used as the neutralizing solution (for example, Example 2-1) than in the case where a neutralizing solution based on delipidized serum was used as the neutralizing solution (for example, Example 2-2), it was possible to further shorten the acid treatment time.

### <Comparison 2 of Neutralizing Solution in Pretreatment of Urine Sample>

### (Reference Example 3)

### [Buffer Neutralizing Solution]

A randomly collected urine sample of volunteer E was used as a urine sample (sample E). The urine sample and hydrochloric acid of 0.2N were mixed such that the volume ratio (urine sample: hydrochloric acid) became 1:2, followed by incubation at 30°c for 18 hours to conduct acid treatment. Subsequently, a buffer having a MOPS concentration of 2M (2M MOPS, 150 mM NaCl, 1% HPβCD, 0.025% Lipidure 802 (manufactured by NOF Corporation), pH 7.3, pKa=7.2) was mixed as a neutralizing solution with the solution after the acid treatment (acid-treated solution, 0.13N hydrochloric acid) such that the volume ratio (acid-treated solution:neutralizing solution) became 1:9 to obtain a measurement sample (neutralization-treated solution) . The steroid measurement was conducted in the same manner as in Reference Example 1-1 except that the measurement sample obtained was used.

### [Delipidized Serum Neutralizing Solution]

In addition, measurement samples were prepared in the same manner as described above except that the neutralizing solution was changed to delipidized serum ("DDC Mass Spect Gold", steroid hormone and cholesterol·TG-free serum, manufactured by VERITAS Corporation), and steroid measurement was conducted on each measurement sample.

### (Comparative Example 3, Example 3)

### [Buffer Neutralizing Solution]

The urine sample (sample E) and hydrochloric acid of 0.2N (Comparative Example 3), 1N (Example 3-1), 2N (Example 3-2), or 3N (Example 3-3) were mixed such that the volume ratio (urine sample:hydrochloric acid) became 1:2, followed by incubation at 37°C for 6.5 minutes to conduct acid treatment. Subsequently, a buffer having a MOPS concentration of 2M (2M MOPS, 150 mM NaCl, 1% HPβCD, 0.025% Lipidure 802 (manufactured by NOF Corporation), pH 7.3, pKa=7.2) was mixed as a neutralizing solution with each solution after the acid treatment (acid-treated solution, 0.13N hydrochloric acid (Comparative Example 3), 0.67N hydrochloric acid (Example 3-1), 1.33N hydrochloric acid (Example 3-2), or 2N hydrochloric acid (Example 3-3)) such that the volume ratio (acid-treated solution:neutralizing solution) became 1:9 to obtain a measurement sample (neutralization-treated solution). The steroid measurement was conducted in the same manner as in Reference Example 1-1 except that the measurement sample obtained was used.

### [Delipidized Serum Neutralizing Solution]

In addition, measurement samples were prepared in the same manner as described above except that the neutralizing solution was changed to delipidized serum ("DDC Mass Spect Gold", steroid hormone and cholesterol·TG-free serum, manufactured by VERITAS Corporation), and steroid measurement was conducted on each measurement sample.

Table 2 below shows the measured value averages in Reference Example 3, Comparative Example 3, and Examples 3-1 to 3-3 and the ratios of the measured value averages (measured value vs control (%)) when the respective neutralizing solutions in Comparative Example 3 and Examples 3-1 to 3-3 were used where the measured value averages when the respective neutralizing solutions in Reference Example 3 were used were each regarded as 100% (control).

**[Table 2]**

| | | Reference Example 3 (control) | Comparative Example 3 | Example 3-1 | Example 3-2 | Example 3-3 |
|---|---|---|---|---|---|---|
| Concentration of HCl | | 0.2N | 0.2N | 1N | 2N | 3N |
| Acid treatment conditions | | 30°C 18h | 37°C 6.5min | | | |
| Measure value average (pg/mL) | Buffer neutralizing solution | 96.7 | 8.1 | 26.8 | 58.3 | 87 |
| | Delipidized serum neutralizing solution | 99.6 | 8.3 | 27.2 | 59.3 | 74.3 |
| Measure value vs control | Buffer neutralizing solution | 100% | 8% | 28% | 60% | 90% |
| | Delipidized serum neutralizing solution | 100% | 8% | 27% | 60% | 75% |

As shown in Table 2, it was confirmed that measurement results similar to those in the case where acid treatment was conducted under the conventional long-time condition (for example, Reference Example 3) were obtained by setting the normality of acid in the acid-treated solution to 0.6N or more (for example, Example 3-1 to 3-3), even when the time for acid treatment was shortened to 6.5 minutes, and it was confirmed that similar behaviors were exhibited in the case where a buffer was used as the neutralizing solution and the case where delipidized serum was used as the neutralizing solution at least up to around 2N. However, in the case where delipidized serum was used as the neutralizing solution, there was a tendency that when the normality of acid in acid treatment was further increased, the value of the measured value average decreased as compared with the case where a buffer was used as the neutralizing solution.

### < Comparison 3 of Neutralizing Solution in Pretreatment of Urine Sample>

### (Reference Example 4)

### [Buffer Neutralizing Solution]

A randomly collected urine sample of volunteer F was used as a urine sample (sample F). The urine sample and hydrochloric acid of 0.2N were mixed such that the volume ratio (urine sample:hydrochloric acid) became 1:2, followed by incubation at 30°C for 18 hours to conduct acid treatment. Subsequently, a buffer having a MOPS concentration of 2M (2M MOPS, 150 mM NaCl, 1% HPβCD, 0.025% Lipidure 802 (manufactured by NOF Corporation), pH 7.3, pKa=7.2) was mixed as a neutralizing solution with the solution after the acid treatment (acid-treated solution, 0.13N hydrochloric acid) such that the volume ratio (acid-treated solution:neutralizing solution) became 1:9 to obtain a measurement sample (neutralization-treated solution) . The steroid measurement was conducted in the same manner as in Reference Example 1-1 except that the measurement sample obtained was used.

### [Delipidized Serum Neutralizing Solution]

In addition, measurement samples were prepared in the same manner as described above except that the neutralizing solution was changed to delipidized serum ("DDC Mass Spect Gold", steroid hormone and cholesterol·TG-free serum, manufactured by VERITAS Corporation), and steroid measurement was conducted on each measurement sample.

### (Example 4)

### [Buffer Neutralizing Solution]

The urine sample (sample F) and hydrochloric acid of 1N were mixed such that the volume ratio (urine sample:hydrochloric acid) became 1:2, followed by incubation at 37°C for 10 minutes, 30 minutes, 1 hour, 2 hours, or 3 hours to conduct acid treatment. Subsequently, a buffer having a MOPS concentration of 2M (2M MOPS, 150 mM NaCl, 1% HPβCD, 0.025% Lipidure 802 (manufactured by NOF Corporation) , pH 7.3, pKa=7.2) was mixed as a neutralizing solution with each solution after the acid treatment (acid-treated solution, 0.67N hydrochloric acid) such that the volume ratio (acid-treated solution:neutralizing solution) became 1:9 to obtain a measurement sample (neutralization-treated solution). The steroid measurement was conducted in the same manner as in Reference Example 1-1 except that the measurement sample obtained was used.

### [Delipidized Serum Neutralizing Solution]

In addition, measurement samples were prepared in the same manner as described above except that the neutralizing solution was changed to delipidized serum ("DDC Mass Spect Gold", steroid hormone and cholesterol·TG-free serum, manufactured by VERITAS Corporation), and steroid measurement was conducted on each measurement sample.

FIG. 3 shows relations between the acid treatment time and the ratios of the measured value averages in Example 4 (Vs the measured value of acid treatment with 0.2N HCl for 18h at 30°C (%)) where the measured value average in Reference Example 4 was regarded as 100% (control).

As shown in FIG. 3, it was confirmed that similar behaviors were exhibited in the case where a buffer was used as the base of the neutralizing solution and the case where delipidized serum was used as the base of the neutralizing solution, and it was possible to sufficiently shorten the time for acid treatment in either case, at least in the case where the normality of acid in acid-treated solution was 0.67N.

### [Industrial Applicability]

As described above, the present invention makes it possible to provide a method for measuring a steroid in a urine sample as well as a kit for measuring a steroid in a urine sample and a neutralizing solution for use in the method that are capable of pretreating a urine sample in a shorter time than the conventional methods. In addition, this also makes it possible to consistently automate operations from pretreatment of a urine sample to completion of a measurement. Since steroid activities relate to various diseases, the present invention not only can be used in research but also can greatly contribute to diagnoses of diseases.

## Claims

1. A method for measuring a steroid in a urine sample, comprising:
an acid treatment step of mixing and reacting an acid solution with a urine sample such that a normality of acid becomes 0.6 to 4N to obtain an acid-treated solution;
a neutralization treatment step of mixing and reacting the acid-treated solution and a neutralizing solution to obtain a neutralization-treated solution; and
a measurement step of mixing the neutralization-treated solution and an antibody capable of specifically binding to a steroid to measure the steroid.

2. The method for measuring a steroid in a urine sample according to claim 1, wherein
pH of the acid-treated solution at 20 to 40°C is 1.0 to 6.0.

3. The method for measuring a steroid in a urine sample according to claim 1 or 2, wherein
a reaction time in the acid treatment step is 1 to 60 minutes.

4. The method for measuring a steroid in a urine sample according to any one of claims 1 to 3, wherein
a normality of acid of the acid solution is 1 to 6N.

5. The method for measuring a steroid in a urine sample according to any one of claims 1 to 4, wherein
pH of the neutralization-treated solution at 25°C is 4.0 to 10.0.

6. The method for measuring a steroid in a urine sample according to any one of claims 1 to 5, wherein
the neutralizing solution contains at least one selected from the group consisting of Good's buffer agents, Tris buffer agents, sodium carbonate, sodium bicarbonate, sodium borate, and basic substances.

7. The method for measuring a steroid in a urine sample according to any one of claims 1 to 6, wherein
pKa of the neutralizing solution at 20°C is 6 to 8.

8. A kit for use in the method for measuring a steroid in a urine sample according to any one of claims 1 to 7, comprising:
at least one selected from the group consisting of
an acid solution having a normality of acid of 1 to 6N,
a neutralizing solution containing at least one selected from the group consisting of Good's buffer agents, Tris buffer agents, sodium carbonate, sodium bicarbonate, sodium borate, and basic substances, and
an antibody capable of specifically binding to a steroid.

9. A neutralizing solution for measuring a steroid in a urine sample for use in the method for measuring a steroid in a urine sample according to any one of claims 1 to 7, comprising:
at least one selected from the group consisting of Good's buffer agents, Tris buffer agents, sodium carbonate, sodium bicarbonate, sodium borate, and basic substances.
